# EUROPEAN PATENT APPLICATION

(11) **EP 2 138 185 A1**
(43) Date of publication of application: **30.12.2009**
(21) Application number: 08011674.2
(22) Date of filing: 27.06.2008
(51) Int. Cl.: A61K 38/12, C12N 15/01, G01N 33/68

(54) **System for the determination of molecules altering the function of interferon, method therefor and compounds altering interferon activity**

(71) Applicant: Helmholtz-Zentrum für Infektionsforschung GmbH, 38124 Braunschweig (DE)
(72) Inventor: Köster, Mario, 38106 Braunschweig (DE); Pulverer. Julia, 31141 Hildesheim (DE); Sasse, Florenz, 38124 Braunschweig (DE); Hauser, Hansjörg, 38302 Wolfenbüttel (DE)
(74) Representative: Kröncke, Rolf

(57) **Abstract**

The present invention relates to a system and a method for the determination of molecules and compounds altering the function of interferon, in particular, type I and type II interferon. In particular, said method and system comprise a cell system and its use wherein said cell system at least partially contains large parts of exogenous chromosomal DNA, namely interferon responsive DNA promoter elements operably linked with a nucleic acid sequence encoding a reporter or marker molecule. In another aspect, cyclic peptides are provided identified as molecules altering the interferon activity, in particular, inhibiting interferon response.

## Description

The present invention relates to a system and a method for the determination of molecules and compounds altering the function of interferons, in particular, type I and type II interferon. In particular, said method and system comprise a cell system and its use wherein said cell system at least partially contains large parts of exogenous chromosomal DNA, namely interferon responsive DNA promoter elements operably linked with a nucleic acid sequence encoding a reporter or marker molecule. In another aspect, cyclic peptides are provided identified as molecules altering the interferon activity, in particular, inhibiting interferon response.

### Background of the invention

Interferons (IFNs) as a typical representative of cytokines and chemokines produced after activation of the host defence in mammals elicit at broad range of biological effects via receptor binding and subsequent activation of different signal transduction pathways. IFNs exhibit a plethora of different effects mediated by reprogramming gene expression within their target cells. By activation of a wide set of genes, IFNs can act either direct (in an auto- and paracrine manner) or indirect through innate and adaptive immune responses on cells. In addition to these antiviral and immunoregulatory effects, IFNs exhibit inflammatory, antimicrobial, immunoregulatory, pro- and anti-apoptotic, as well as activities regulating proliferation- and differentiation.

The interferon system comprises a group of pleiotropic cytokines which are involved in numerous immune interactions. During infectious processes, IFNs contribute to the induction as well as regulation of innate and adaptive immunity. These cytokines were first described by Isaacs and Lindenmann for their activities in 1957 and got named "interferon" (IFN) based on the latin words *inter* (between) and *ferire* (to strike). Nine years later the only member of type II IFN, i.e. IFN-γ, was first published and described as an "IFN-like virus inhibiting protein produced by human T-cells".

However, it required more than a decade until introduction of cloning and purification techniques led to production of greater amounts of IFNs and successive research on these proteins. Subsequently, the ingrained concept of IFNs only exhibiting antiviral activities was extended by their important roles in controlling other pathogenic infections as well as having impact in cell metabolism and initiation of adaptive immunity.

According to their genomic loci, amino acid sequences and their signalling via distinct receptors, IFNs are nowadays classified into three groups, namely type I, II and III.

Type I IFNs present the greatest class, encoded by distinct intron-less (except for IFN-κ) genes clustered on chromosome 9 in humans or in regions of conserved synteny on mouse chromosome 4. Among mammals, the number of type I IFN genes is variable. Most important for viral resistance are the 13 to 14 different IFN-α isoforms, as well as one IFN-β protein. All of these cytokines express slightly different activity profiles regulated by stimulus, cell type and donor.

Within one species all type I IFNs share homologies between 30 to 85 % and exhibit relatively high antiviral potencies. Although qualitative as well as quantitative differences in their biological effects exist, their origin and maintenance through evolution are not revealed by now. Other less well known and characterized representatives of type I IFNs are IFN-ε, -κ, -δ -v, -τ, -ω and -ξ/limitin.Whereas IFN-δ (pigs), -τ (cattle), -v (cats) and limitin (mice) are not found in humans, IFN-ω exhibits same antiviral effects like IFN-α/β and is found in humans and sheep, but not in mice. Limitin has antiviral, antitumoral as well as immunomodulatory functions like IFN-αs, but shows attenuated lymphomyelosuppressive potency. In contrast, IFN- ε was found to be expressed in ovaries and uterus but not in tissues of hematopoietic origin. All type I IFNs belong to the helical cytokine family with secondary structures of five α-helix bundles stabilised by two disulphide bonds and bind to the same receptor composed of IFNAR1 and IFNAR2. As IFN-αs and -β are the most prominent and - in terms of viral infections - important members of this group, successive information on type I IFNs will in most instances refer to these cytokines.

On the other hand, there is only one member of the type II IFN class, namely IFN-γ. Structurally, this protein does not show significant homology to type I IFNs, but due to the original IFN-definition of "interfering" with viral replication, it was classified as such. IFN-γ is nowadays particularly known for its immunostimulatory and immunomodulatory functions. Besides, this cytokine is crucial for immunity against viral and intracellular bacterial infections and plays an important role in autoimmunity and tumour surveillance (Schoenborn, J. R. and C. B. Wilson (2007). Adv Immunol. 96:41-101). The gene encoding this cytokine is located on the human chromosome 12, in mice on number 10.

The third group of IFNs is composed of the recently identified proteins IFN-λ1, -λ2 and -λ3 also referred to as IL-29, IL-28A and IL-28B, respectively. These cytokines are structurally and genetically related to cytokines of the IL-10 family, but display close activities to type I IFNs. IFN-λs are all encoded by separate intron-containing genes closely linked on human chromosome 19. While the murine IFN-λ1 1 is a pseudogene, IFN-λ2 and -λ3 genes encode for glycosylated proteins.

A common feature of all members of the IFN family is their secretion of cells after stimulation and subsequent binding to their cognate receptors. All classes interact with their type-specific receptor complexes and ligand-receptor binding triggers the activation of a main signalling pathway leading to transcriptional induction of several hundred genes.

For example, three IFN-induced antiviral pathways have been established so far, comprising PKR, the 2'-5'oligoadenylate synthetase (OAS)/RNaseL system and the Mx proteins. After its activation by viral RNA the serine/threonine kinase PKR phosphorylates one subunit of the translation initiation factor 2 (eIF2α), which results in downregulation of translation leading to detrimental effects on the cell. The immunomodulatory functions of IFNs comprise the upregulation of cell surface major histocompatibility complex (MHC) protein expression, activation of NK cell proliferation and cytotoxicity, the control of DC maturation and activation as well as their enhancing or suppressive effects on B- and T-cells. Another important activity of IFNs is their antiproliferative as well as apoptosis-inducing impact on cells. However, as also true for other IFN activities, IFNs can generally be inhibitors of proliferation, but can also stimulate proliferation of memory T-cells.

Although IFNs are central mediators of both innate and adaptive immune responses, there is also a pathogenic role of these cytokines known. For instances, IFNs were shown to be negatively involved in the pathogenesis of lipopolysaccharide-induced endotoxic shock (Karaghiosoff M, et al., (2003). Nat Immunol. 4:471-477). Moreover, in some systemic and organ-specific autoimmune diseases, for example systemic lupus erythematosus (SLE) and diabetes mellitus (IDDM), production of IFNs is believed to lead to detrimental effects on the organism (Theofilopoulos AN, et al., (2005). Annu Rev Immunol. 23:307-336). Furthermore, disease progression in HIV-patients was found to be associated with elevated IFN-levels (Herbeuval, J. P. and G. M. Shearer (2007). Clin Immunol. 123:121-128).

IFNs have also been reported to be produced in the absence of infections and have important effects, e.g. in developmental processes (Takayanagi H, et al., (2002). Nature 416:744-749). It is therefore possible that other activities have not yet been recognized.

As mentioned before, IFNs exhibit various biological activities and are therefore of major relevance for therapeutically use. Although hopes that interferons might be the miracle drug for viral infections as well as cancer did not prove true, IFNs are now widely used for treatment of hepatitis C, relapsing forms of multiple sclerosis and some cancers. IFN treatment is unfortunately not effective in all patients and due to adverse effects from systemic IFN-administration its therapeutic efficacy and clinical utility is limited. In 1986, IFN-α2was the first biotherapeutic approved by the U.S. Food and Drug Administration and thus paved the way for many others. Despite the enormous potential of IFNs their use as therapeutics is limited since dose-dependent side effects could be revealed. These include fever, fatigue, malaise, anorexia and many others. Though, to date, IFN-αs are approved for treatment of hairy cell leukaemia, malignant melanoma, follicular lymphoma, condylomata acuminata (genital warts), AIDS-related Kaposi sarcoma as well as hepatitis B and C infections. On the other hand IFN-β is used in the therapy of relapsing forms of multiple sclerosis and IFN-γ for chronic granulomatous disease and malignant osteropetrosis.

Several techniques for engineering transgenic animals are nowadays available, accessing the germline genome through gametes, zygotes and embryonic stem (ES) cells. In this respect the choice between random integration and homologous recombination has to be made. Random integration of small transgenes might lead to a so-called position effect, where the transgene expression is influenced by the local environment at the integration site. However, this can be overcome by employing bacterial artificial chromosomes (BACs). As an alternative to yeast artificial chromosomes (YACs) BACs allow easy manipulation of large DNA sequences. These bacterial artificial chromosomes (BACs) can propagate up to 300 kb of genomic sequences and replicate at low copies in a recombination deficient and RecBCD exonuclease-deficient E.coli host strain. Since significant progress has been made for rapid modification of BACs they are nowadays commonly used. Moreover, in most genome projects these cloning vectors are used for sequencing, which result in a large number of now available BACs containing genomic segments of several species.

Due to the great size of BACs modification techniques are rather based on homologous recombination events than on cloning systems with restriction enzymes, since unique enzyme cleavage sites are rare. Several methods have been described and first reports were based on shuttle vectors containing the gene modification flanked by BAC homologous regions of around 0.5 - 1 kb as well as the bacterial recombinase RedA. A second approach was the introduction of linear fragments flanked by around 50 bp-long homologous sequences into BAC-containing bacteria. These recombination events are mediated by inducible phage-derived recombination enzymes encoded on separate vectors. The technique was termed "Recombineering" according to recombination-mediated genetic engineering.

First publications demonstrating the use of recombinant BACs for generation of transgenic animals and subsequent germline-transmission of the transgene was done in 1997 (Antoch, M. P., E. J. Song, et al. (1997). Cell 89:655-667). This paved the way for generation of many other BAC transgenic animals. The use of marker proteins into endogenous loci of vertebrates and invertebrate genes has not generally altered expression patterns and data evidenced that expression of large DNA transgenes can accurately reflect pattern of transcription of the endogenous chromosomal gene.

As illustrated type I and III IFNs are central players in innate immune responses towards a variety of pathogens. These cytokines exhibit a plethora of effects mediated by activities of interferon-stimulated genes (ISG). Since most studies carried out for unravelling these immune responses were done in vitro, the identity of cell types eliciting coordinated antiviral, antiproliferative or immunomodulatory functions of these proteins in vivo are mostly unknown. Thus, the object of the present invention aims to advance understanding of these immune reactions leading to type I IFN expression and subsequent induction of their versatile responses in an autocrine and paracrine manner. For example, a transgenic mouse model allows to elucidate IFN-α/β activities.

Further, Type II IFN plays a significant effect in T cell regulation and is therefore a potential target for treatment of autoimmune diseases.

Another object of the present invention is the provision of reporters for monitoring type I as well as type II IFN activities in respect to improved specificity and sensitivity for further use in cell culture systems. Moreover, available bioassays for quantification of type I IFNs amounts harbour several disadvantages.

That is, presently various bioassays are available based on the one hand on cell systems containing IFN responsive DNA promoter sequences of limited size of less than 3 kb present in a plasmid. However, sensitivity and specificity of said systems are low. On the other hand, for the quantification of IFN an antiviral assay which is based on the protective effect of IFN against viruses such as vesicular stomatitis virus (VSV) is used. The ability of a sample to reduce lysis of IFN susceptible cells infected with VSV is assessed visually by light microscopy and compared to an IFN standard solution. This method is highly subjective and does not give a quantitative read out system.

Finally, ELISA systems for the quantification of IFNs are on the market. However, this non-bioassay does not determine the amount of biologically active IFN but of the molecule as such regardless of its activity.

Therefore a novel system needs to be established and validated allowing quantification of IFN with high sensitivity and specificity. IFNs can elicit a broad spectrum of biological activities and are therefore of great importance in therapeutic use. However, there is also a pathogenic role of IFNs known, especially in diseases of endotoxic shock and systemic lupus erythematosus (SLE). Hence, there is an ongoing need for agonists, enhancers as well as antagonists of the interferon signalling pathway.

From various bacteria, like Myxobacteria, secondary metabolites have been described having different capabilities and biological activities. For example, from myxobacteria a family of cyclic peptides is known, the so called vioprolides. Said cyclic peptides have been isolated from strain CB vi35 of *Cystobacter violaceus* as described e.g. in Schummer D. et al, 1996, Liebigs Ann. Chem. 971. They display some unusual chemical structures. In particular, some of the vioprolides have a 4-methyl-azeditine carboxyl acid replacing a proline in two of the described variants, a homoproline, replacing a second proline in two of the variants; and an L-glycerid acid interrupting the amino acid chain in one place. For the vioprolides anti-fungal and highly cytotoxic activities have been described.

In view of the above, there is an ongoing need for systems allowing the determination of molecules altering the function of IFN and, thus, modulating IFN activity in vivo. Further, there is an ongoing need for methods allowing the determination including screening, identification, validation, and (semi)-quantification of compounds and molecules altering the function of IFN, in particular, of type I, type III and/or type II interferon.

The present invention addresses the above needs and provides systems and methods useful for the determination of molecules or compounds altering the function of IFN. Moreover, a class of molecules is provided demonstrating an inhibitory effect on IFN activity, in particular of type I and/or type II IFN activity. Further, the present invention provides a method and system for the quantification of IFN.

Finally, the invention provides new uses of compounds being effective to inhibit interferon type I activity in subjects suffering from various diseases, disorders, or conditions characterized in having a detrimental interferon type I/type III or interferon type II activity. Such molecules would be of beneficial use in a variety of applications, including prevention or treatment of septic shock syndrome, or autoimmune diseases, in particular, of systemic lupus erythematosus and diabetes mellitus.

### Summary of the invention

The present invention relates to a system for the determination of molecules or compounds altering the function of interferon (IFN) comprising a cell system at least partially containing exogenous chromosomal DNA delivered by a recombinant vector containing interferon responsive DNA promoter elements having a size of at least 3 kb 5' upstream of DNA binding elements conferring minimal responsiveness to interferon stimulation, in particular, interferon type I/type III and type II stimulation, operably linked with a nucleic acid sequence encoding a reporter or marker molecule, into cells of the cell system.

Moreover, the present invention relates to a system wherein said recombinant vector is a recombinant YAC or BAC, in particular, a recombinant YAC or BAC containing the Mx1or Mx2 gene locus when determining molecules altering the function of interferon type I or the Gbp gene locus for analyzing the function of interferon type II.

Preferably, said cell system is a transgenic mouse.

Furthermore, the present invention relates to methods for determining molecules or compounds altering the function of interferon using the cell system according to the present invention, in particular, using a transgenic mouse model.

Moreover, the present invention relates to new pharmaceuticals useful for the prevention or treatment of septic shock syndrome or autoimmune diseases, in particular, for the prevention or treatment of organo specific autoimmune diseases, comprising the administration of cyclic peptides according to general formula I, thus, altering, in particular, inhibiting interferon type I and/or interferon type II activity.

Preferably, said pharmaceutical composition comprises vioprolide in combination with other active ingredients useful for the treatment of the respective diseases.

Finally, the present invention relates to methods for preventing or treating septic shock syndrome or autoimmune diseases to an individual comprising administering the molecules determined with the method according to the present invention or the cell system according to the present invention and, in particular, the cyclic peptides of general formula I, optionally, with further active ingredients useful for the treatment of the respective diseases.

### Brief description of the drawings

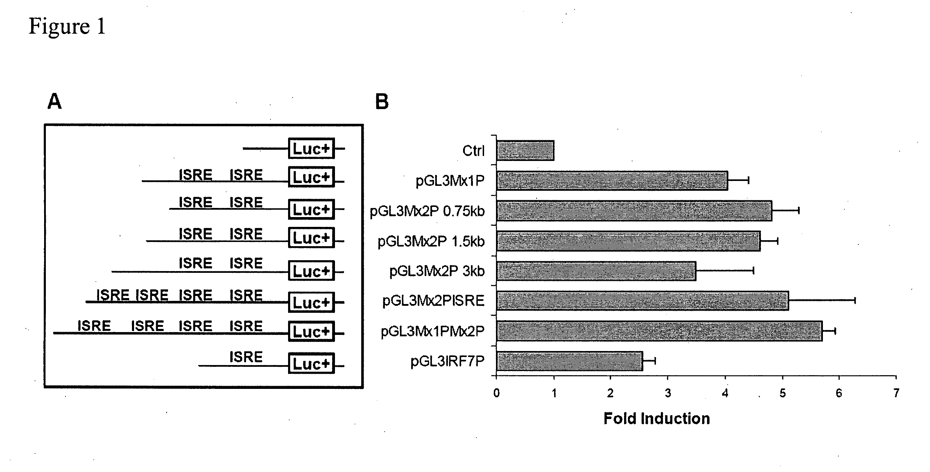

In figure 1, the type I/II IFN-responsive promoter constructs are shown.
Reporter plasmids containing Firefly luciferase under control of different promoter fragments of the Mx1, Mx2 and Irf7 gene as well as artificial promoter constructs were transiently expressed in NIH/3T3 cells. In figure 1A a schematic overview of IFN responsive elements (ISRE) in promoter constructs used is provided. In figure 1B inducibility of luciferase expression was validated by treatment of cells with IFN-β (500 U/ml). After 24 hours luciferase activity was analysed by luminometric assay. Fold induction represents relative luminescence units (RLU) of IFN-stimulated compared to untreated cells. Data represent mean ± SD of two independent experiments (n=2).

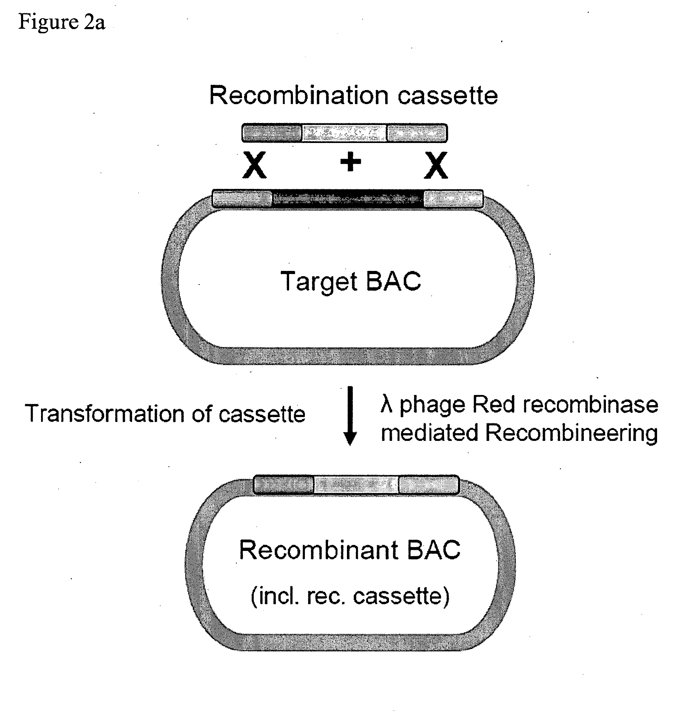

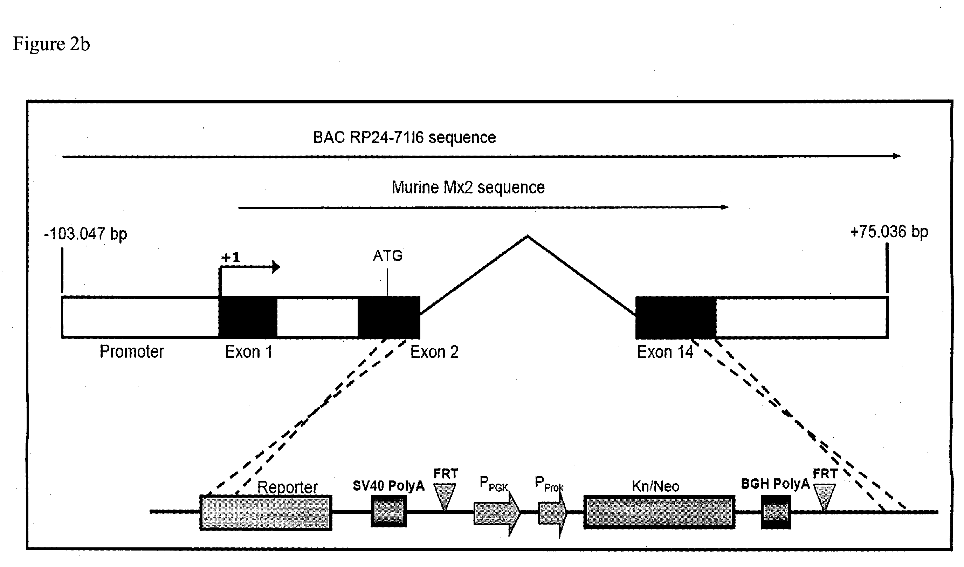

Figure 2 shows the recombineering steps and strategy for obtaining BAC useful as vectors according to the present invention.
In figure 2a the recombineering Strategy using the phage λ Red recombinase system is illustrated. Recombination cassette is generated by PCR using primers with flanking sequences homologous to the BAC. Homologous regions are displayed in different grey scales. The recombination system needs to be introduced into bacteria harbouring the target BAC. Bacterial strains containing both the BAC and enzymes mediating homologous recombination are then transformed with the recombination cassette. Correct recombinants can then be selected with markers and checked with PCR and diagnostic digest. In figure 2B, the recombination cassette is shown which contains a gene for a bioluminescent (Firefly Luciferase) or fluorescent (tdTomato) reporter followed by SV40 PolyA signal and FRT-flanked fragment of prokaryotic and eukaryotic (PGK) promoter elements controlling expression of the kanamycin/neomycin phosphotransferase gene followed by the PolyA signal of the bovine growth hormone gene (BGH PolyA). The Mx2 ORF located on the BAC RP24-71I6 was replaced with the recombination cassette as depicted using a phage-derived recombination system.

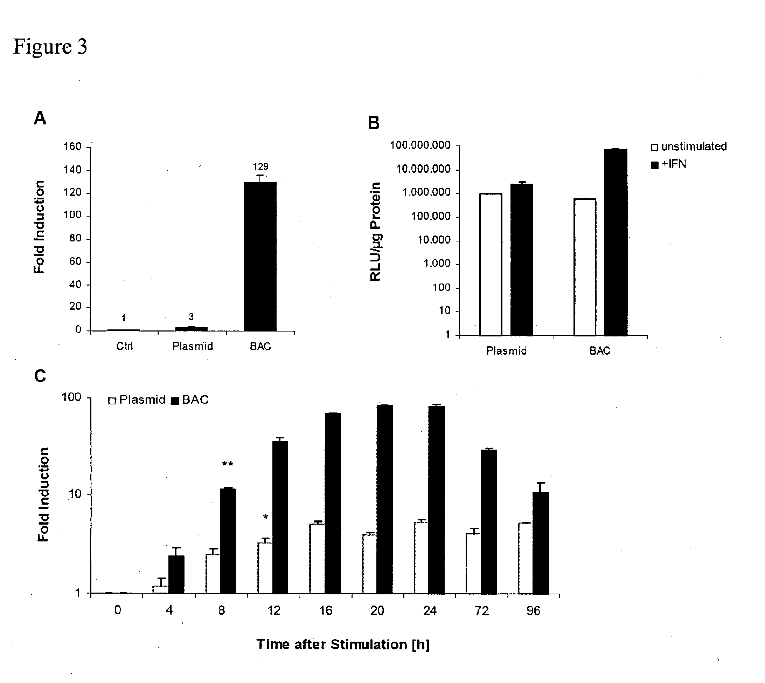

Figure 3 provides BAC- vs. Plasmid encoded reporter constructs. Cell pools of NIH/3T3 stably transfected by electroporation with the BAC Mx2Luc ("BAC") or the plasmid pGL3-Mx2P 0.75kb ("Plasmid") were used for comparison of IFN responses. In figure 3A fold induction resembling RLU of IFN-(β-stimulated (500 U/ml) compared to uninduced cells is displayed. As control the promoterless pGL3-basic vector (Promega) was used. Figure 3B shows RLUs calculated to one µg of protein detected by BCA assay. RLUs were assessed in both unstimulated and IFN-β-stimulated (500 U/ml) cells containing the plasmid (pgL3Mx2P0.75kb) or BAC (Mx2Luc) based reporter. In figure 3C temporal regulation (0 - 96 h) of the plasmid (pgL3Mx2P0.75kb) or BAC (Mx2Luc) based reporter systems after stimulation with 500 U/ml of IFN-β is shown. Fold induction resembles detected RLUs compared to uninduced state. P-values were calculated by student's t test (* p=0.008, **p=0.017). Data represent mean ±SD of two experiments (n=2).

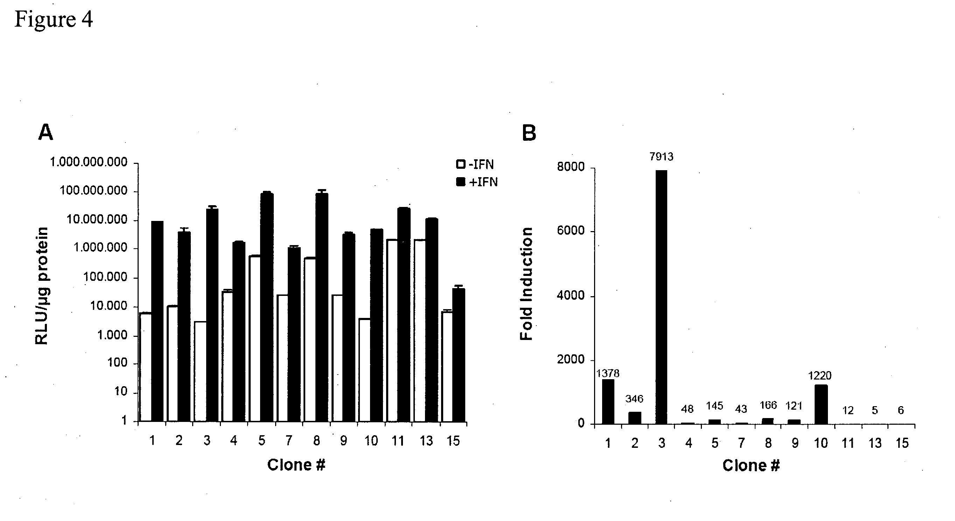

Figure 4 shows the analysis of BAC-reporter containing cell clones. NIH/3T3 cells were electroporated with recombinant BAC Mx2Luc2 and cell clones stably expressing the BAC were obtained by G418-selection. Cells were expanded and subjected for further analysis to their response to IFN-β. Cells were seeded on 6-well plates and stimulated wit 500 U/ml of IFN-β for 24 hours (+IFN) or left untreated (-IFN). Luciferase Activity was analysed by luminometric assay (n=2). In figure 4A relative luciferase units (RLU) were calculated to protein amount, which was obtained by BCA-assay. Results of each obtained cell clone are displayed. In figure 4B fold induction of RLU obtained after IFN-stimulation compared to untreated cells is displayed.

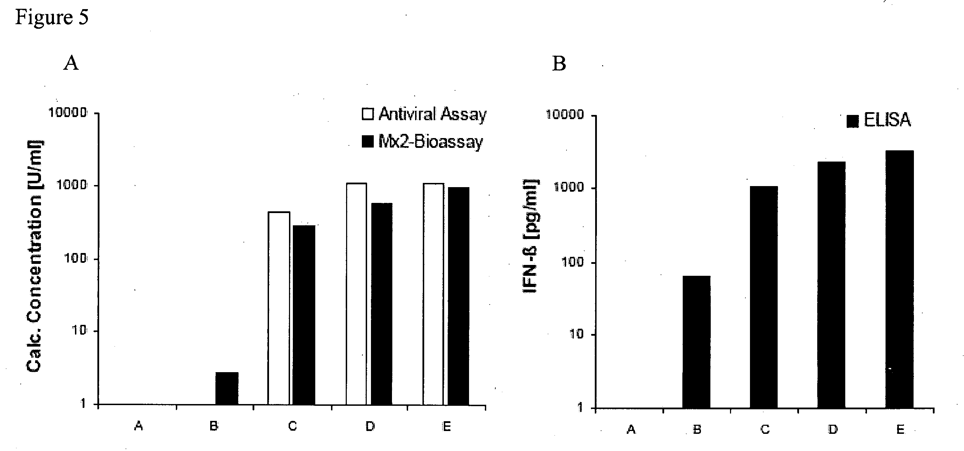

In figure 5 a comparison of Mx2-bioassay to the antiviral IFN-assay is provided. Five samples of unknown IFN-concentrations were employed to compare the new established Mx2-bioassay with the common used antiviral assay to determine IFN-concentrations. In figure 5A an antiviral assay was performed as described in Materials and Methods. Samples were applied onto LMTK cells in two different dilutions: 1.row: undiluted, 2.row: 1:10 diluted in culture media. For control (Ctrl) 500 U/ml IFN-β were applied onto the cells. Image represents results obtained by two independent experiments. Figure 5B shows the calculated concentrations of samples (A-E) obtained by antiviral assay and the Mx2-bioassay. To determine IFN-concentrations with Mx2-bioassay the IFN-reporter cells, containing the recombinant BAC Mx2Luc2, were seeded on 24-well plates (2.104 cells / well) and incubated with 20% of IFN-samples overnight. For quantification a standard curve of different IFN-β concentrations (0, 10, 25, 50, 100 and 250 U/ml) was additionally applied. Unknown IFN-concentrations were calculated by correlating obtained RLUs to employed standard curve. In figure 5C IFN-β concentrations [pg/ml] obtained by an IFN-β specific ELISA (PBL) are shown.

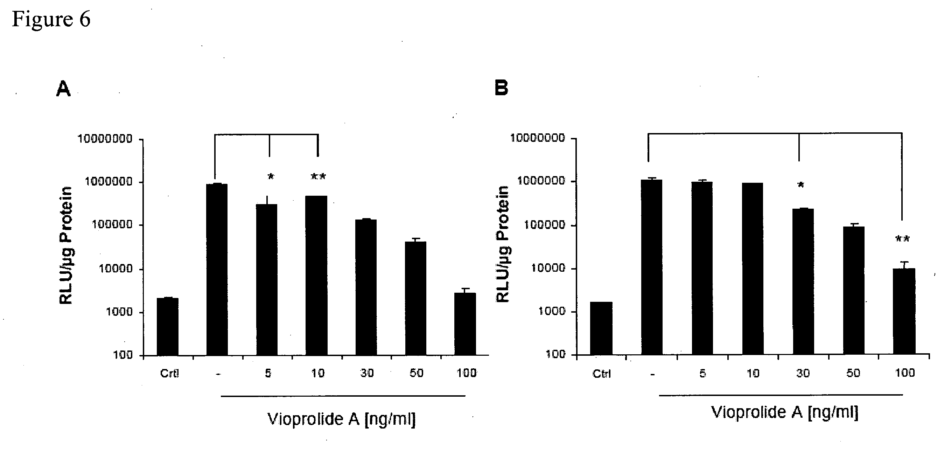

In figure 6 the Inhibitory effect of Vioprolide A on the type I IFN response is demonstrated. In figure 6A the type I IFN reporter cells were pretreated with different concentrations of Vioprolide A (0, 5, 10, 30, 50 or 100 ng/ml). After pre-treatment for two hours cells were stimulated with IFN-β (500 U/ml) in the continuous presence of the indicated Vioprolide concentrations. In figure 6B type I IFN reporter cells were pretreated with IFN-β (500 U/ml). After four hours cells were additionally stimulated with different concentrations of Vioprolide A (0, 5, 10, 30, 50 or 100 ng/ml). Untreated cells were used as control (Crtl). Luciferase activity was measured after 24 hours and relative luminescent units (RLU) were calculated to one µg of protein detected by Bradford assay. Mean values ± SD are displayed (n=2). * p<0.05 and **p<0.001 for Student's t test compared to IFN-stimulated cells without Vioprolide treatment.

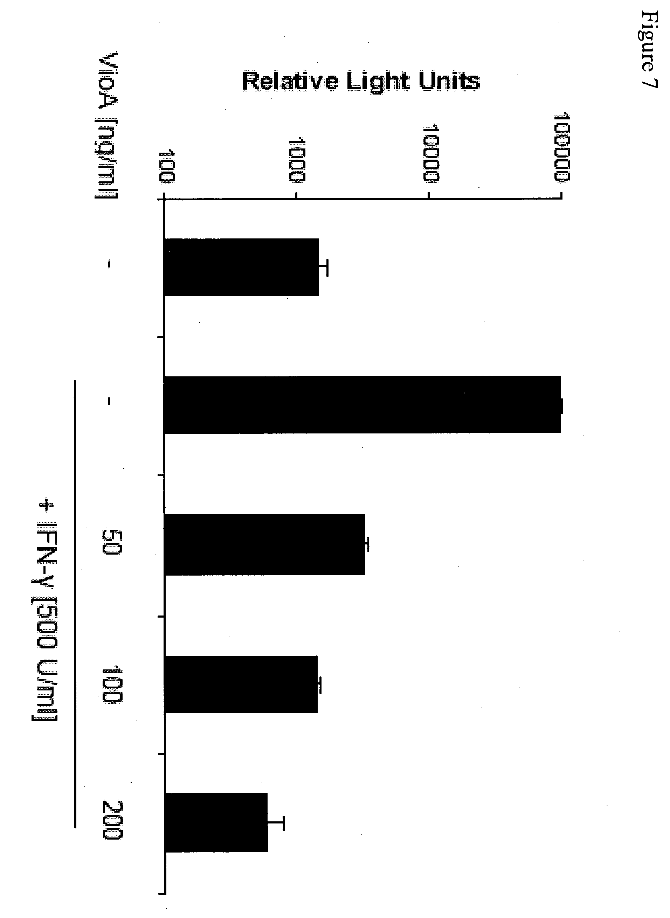

In figure 7 the inhibitory effect of Vioprolide A on the type II IFN response is provided. IFN-γ reporter NIH/3T3 cells harbouring the recombinant BAC Gbp2RLuc were seeded on 96-well-plates and treated with IFN-γ (500 U/ml) or left untreated ("-"). After one hour cells were exposed to different concentrations of Vioprolide A (-, 50, 100 or 200 ng/ml) and incubated overnight. Gbp2-driven Renilla luciferase expression was determined by luciferase assay. Results are expressed as fold induction of basal expression (n=3).

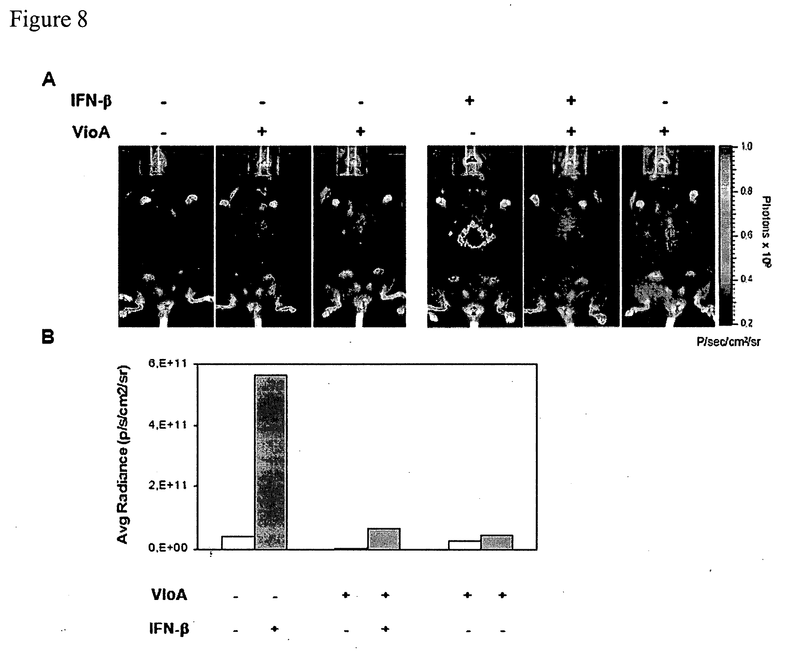

In figure 8 the inhibitory response of Vioprolide A on the type I interferon response is shown. In figure 8A a female type I IFN reporter mice, which contain the IFN-responsive BAC Mx2Luc2, were i.v. treated with 20µg of Vioprolide A (in 100% EtOH). First image was taken immediately after Vioprolide A treatment. After 1 h mice were i.v. injected with 5.000 U/ml IFN-β (PBL). Three hours after IFN-treatment second series of images were taken. For control one animal was only treated with Vioprolide A or with IFN, respectively. Bioluminescent images were obtained using the IVIS200 System (Xenogen). Therefore mice were anaesthetised with 2-2.5% isoflurane (Isoflo, Abbott) and i.p. injected with 100 µl of luciferin (30mg/ml in PBS; Synchem OHG). Rainbow scales indicate number of photons measured per second x cm2 x sr. Figure 8B shows the quantification of the luminescence performed using "Living Image" software and average (avg) radiance calculated as photons per second x cm2 x sr is displayed.

### Detailed description of the present invention

The present invention relates to a system for the determination of molecules altering the function of interferon (IFN) comprising a cell system at least partially containing exogenous chromosomal DNA delivered by a recombinant vector containing interferon responsive DNA promoter elements, having a size of at least 3 kb 5' upstream of the minimal interferon responsive DNA promoter elements conferring responsiveness to type I or type II interferon stimulation, operably linked with a nucleic acid sequence encoding a reporter or marker molecule in at least parts of the cells of the cell system.

As used herein, the terms "disease", "disorder", "pathology" and "condition" relates to septic shock syndrome and autoimmune diseases, allergies, or chronic or acute inflammatory processes.

As used herein, the terms "individual" or "subject" which are used herein interchangeably refer to an individual or a subject in need of the therapy or prophylaxis. The terms "subject" or "individual" as used herein include, but are not limited to an organism; a mammal including e.g. a human, a non-human primate (e.g. baboon, orang-utan, monkey, rodent, like mouse or rat, pig, cow, goat, cat, rabbit, guinea-pig, hamster, horse, sheep or other non-human mammals; a non-mammal.

The term "pharmaceutical composition" means a composition suitable for pharmaceutical use in a subject or individual, including an animal or human. A pharmaceutical composition generally comprises an effective amount of an active agent or ingredient and a carrier, including e.g. a pharmaceutically acceptable carrier.

The term "effective amount" means a dosage or amount sufficient to produce a desired result. The desired result may comprise an objective or subjective improvement in the recipient, i.e. a subject or an individual, of the dosage or amount.

A "prophylactic treatment" is a treatment administered to a subject or an individual who does not display signs or symptoms of a disease, pathology, or medical disorder, or displays only early signs of symptoms of disease, pathology or disorders, such that treatment is administered for the purpose of diminishing, preventing, or decreasing the risk of developing the disease, pathology or medical disorder. In this connection, it is noted that vaccination is one form of prophylactic treatment.

A "therapeutic treatment" is a treatment administered to a subject or an individual to display symptoms or signs of pathology, disease, or disorder in which treatment is administered into the subject for the purpose of diminishing or eliminating those signs or symptoms of pathology, disease or disorder.

As used herein, the term "carrier" refers to a diluent, adjuvant, excipient or vehicle.

The term "interferon responsive DNA promoter element" as used herein means any DNA sequence conferring responsiveness to signalling molecules or transcription factors directly or indirectly induced by binding of the different types of IFN to their respective receptor complex. That is the interferon-stimulated response elements (ISRE) or the IFN-gamma activated sequence (GAS) being in the vicinity of the TATA-Box are present. For example, for the Mx2 gene, two ISRE are about 25 bp and about 90 bp, respectively, upstream of the TATA-box. The "interferon responsive DNA promoter elements" encompass at least the ISRE or the GAS element and the TATA-box.

The term "determination of molecules" refers to various possibilities of determination. That is, the term "determination of molecules" includes the identification of molecules, the validation of molecules, the quantification of molecules and the screening of molecules.

The molecules to be analyzed using the system according to the present invention or with the help of the method according to the present invention comprises all types of molecules including small molecules or compounds, nucleic acid based molecules or amino acid based molecules as well as either naturally occurring or synthetic molecules. Said molecules or compounds may be chemical entities, nucleic acid derivatives or amino acid derivatives including mono- or dinucleotides, mono- or dinucleosides, oligonucleotides having a length of 3 to 50 nucleic acids and polynucleotides having a size of more than 50 nucleic acids. In addition, the molecules may be amino acids, dipeptides, oligopeptides or polypeptides wherein oligopeptides includes amino acid molecules encompassing 3 to 50 amino acids while polypeptides include peptides having more than 50 amino acids including proteins.

Said compounds or molecules may be natural occurring molecules or compounds or synthetic molecules or compounds.

The term "altering the function of interferon" refers to the phenomenon of increasing/enhancing or decreasing/lowering the expression of interferon or increasing/enhancing or decreasing/lowering interferon signalling or other metabolic pathways involving interferon in cells, cell systems or individuals or subjects.

That is, the term "altering" encompasses decreasing or increasing interferon expression as well as increasing or decreasing the activity of interferon including binding properties of transcription factors induced directly or indirectly by interferon to interferon responsive elements, in particular, interferon responsive promoter elements wherein interferon or molecules expressed due to interferon interaction as transcription activators.

The term "cell system" as used herein, refers to either in-vitro or in-vivo cell systems, that is, cell systems include individual or subjects as defined above, in particular, animals, like humans, or rodent animals, which may be obtained by genetic engineering methods. On the other hand, the cell system may be a cell culture comprising primary cells or cell lines transduced with the recombinant vector containing exogenous chromosomal DNA containing interferon responsive DNA promoter elements having a size of at least 3 kb 5' upstream of the interferon interacting site of the interferon responsive DNA promoter elements. Said elements are operably linked with a reporter or marker molecule.

Preferably, the cell system applied in the system and the method according to the present invention is a transgenic rodent, in particular a transgenic mouse. Aternatively, a transduced cell line may be used. Examples of suitable cell lines include cell line NIH3T3 Mx2-Luc2 and NIH3T3 Gbp2-RLuc deposited with the DSMZ, Braunschweig, Germany under the Budapest Treaty.

The NIH3T3 Mx2-Luc2 cell line contains the firefly luciferase under control of Mx2 as described in the examples. Transfection was affected by electroporation with BAC Mx2-Luc2 as described herein.

The NIH3T3 Gbp2-RLuc cell line contains the Renilla luciferase under control of the Gbp gene as described below. Transfection of the NIH3T3 cell line was affected by electroporation with BAC Gbp-RLuc following the method as described herein. These two cell lines are in particular useful for conducting the method of the present invention or in the system according to the present invention.

The system according to the present invention allows monitoring type I as well as type II interferon activities, validating and evaluating effects of type I or type II interferon activity and the provision of assays, in particular, bio assays for (semi-)-quantification of interferon amounts overcoming various disadvantage known in the art.

In a preferred embodiment of the system according to the present invention, the recombinant vector integrated into cells of a cell system are recombinant YAC or BAC molecules comprising exogenous chromosomal DNA including interferon responsive DNA promoter elements operably linked with a nucleic acid sequence reporter or marker encoding a molecule. The BAC (bacterial artificial chromosomes) or YAC (yeast artificial chromosomes) offer several advantages, e.g. said systems are quite stable. These vector systems based on the F episome of *E. coli* can accommodate up to several hundred kb DNA. Further, these systems can be manipulated easily and due to its large size all cis- and transregulatory regions of a given gene are usually located on a BAC construct.

That is, the present inventors found that cells in a cell system wherein the cells are at least partly transduced with a recombinant vector containing exogenous chromosomal DNA whereby said DNA contains interferon responsive DNA promoter elements operably linked with a nucleic acid sequence encoding a reporter or marker molecule allow to evaluate effects of molecules or compounds altering the function of interferon, in particular, the type I as well as type II interferon activities. Thus, a system or method having greater specificity and cell activity than system known in the art can be provided. In particular, bioassays based on cell culture systems or based on the use of a transgenic mouse model are in particular envisaged for the validation and quantification of type I or type II interferon amounts. Further, the cell systems allows to screening for and identify molecules altering the function of interferon, namely the ability of interferon to act on interferon responsive DNA promoter elements, thus, altering transcription of DNA linked with said promoter elements.

BACs or YACs can be microinjected by three different forms: as circular supercoiled plasmid, linearised DNA or purified insert. The preferred method, however, is to employ linearised DNA because this minimizes the risk of obtaining undesirable DNA molecules.

In a particular preferred embodiment of the present invention, the interferon responsive DNA promoter element is selected from the Mx1 and the Mx2 gene locus.

As a result of infection studies using Influenza A virus, Mx proteins were discovered for controlling pathogenicity. Today, Mx proteins are considered as key players in IFN-induced antiviral activities and are found in a variety of organisms like yeasts and vertebrates including fish, mice and humans. Mx proteins are characterized as GTPases with high molecular weight. For example, in mice, two distinct Mx genes designated Mx1 and Mx2, are localized on chromosome 16 in close proximity. Since both nuclear and cytoplasmic forms of Mx GTPases exist, molecular mechanisms of their antiviral activities appear to be dependent on their subcellular localization. In humans, two Mx proteins, MxA and MxB, are encoded on chromosome 21, but only MxA which is localized in the cytoplasm exerts antiviral activity. Thus far, their mechanism of action has not been entirely elucidated. Though, Mx proteins were found to bind essential viral components to block their functions. In many studies, stringent control of Mx-induction by type I/III IFNs but not IFN-γ could be demonstrated Haller et al., 2007, Microbes Infect. 9:1636-1643. The promoter region of the Mx2 gene contains two interferon-stimulated response elements (ISREs) in close proximity to the TATA-box. The proximal ISRE site positioned between -69 and -55 is important for the type I interferon dependent induction of transcription (Asano, A., H. K. Jin, et al. (2003).Gene 306:105-113). Hence, Mx proteins were tested as biomarkers to validate activities of recombinant type I IFNs in humans (Files, J. G., J. L. Gray, et al. (1998). J. Interferon Cytokine Res. 18:1019-1024). Besides, their specificity for type I IFNs was also utilised in bioassays (Bollati-Fogolin, M. and W. Muller (2005). J. lmmunol Methods 306:169-175).

These Mx1 and Mx2 gene locus allow identifying the presence and activity of type I interferon in the cell system. In particular, the genes of the interferon responsive Mx (microvirus gene) locus are particularly useful. These genes are selectively induced by type I and type III interferons showing high sequence homologies.

As demonstrated in the examples for the Mx2 gene locus, a generated reporter BAC harbouring the firefly luciferase as the reporter molecule under control of the Mx2 promoter in comparison to a shorter promoter reporter construct with 0.75 kb of the Mx2 promoter region driving expression of firefly luciferase, the BAC based constructs allow a tremendously higher interferon induced luciferase activity, e.g. over 100 fold compared to 3 fold induction levels of the plasmid based reporter.

Thus, the present inventors found that using recombinant vector systems, like the BAC or YAC system comprising a large parts of the interferon responsive DNA elements, namely elements having a size of at least 3 kb 5' upstream of the interferon responsive DNA promoter elements next to the TATA-box, allows a remarkable enhancement of the specificity due to stronger inducibility by interferons and, in addition, demonstrated a lower basal expression level compared to plasmid systems.

Further, it is demonstrated that the BAC based reporter cells exhibit much stronger induction levels which can be observed earlier during culture compared to the plasmid system.

Preferably, the size of the BAC or YAC construct or other recombinant vector is more than 5 kb like more than 10 kb, in particular, more than 30 kb, like exceeding 50 kb of the flanking 5' and 3' DNA of the interferon inducible gene.

The system allows monitoring interferon type II or type I function, either on the activity level or quantitatively, namely the amount of interferon.

For type II interferon, representing another preferred embodiment of the present invention, the promoter element is in particular the Gbp gene locus.

The system allows the determination of molecules or compounds altering the function of interferon, namely, the screening, identification, validation and quantification as well as monitoring of the function and activity of interferon type I and type II.

Another important element of the recombinant vector introduced into the cells is the nucleic acid sequence encoding the reporter or marker molecule operably linked with the interferon responsive DNA promoter element. Said reporter or marker molecule may be any marker or reporter molecule known by the skilled person. Various molecules are known in the art. In particular, said nucleic acid sequence encoding the reporter or marker molecule is selected from the group consisting of bioluminescent, fluorescent, enzymatic and antigenic reporter genes, cell surface markers and selection markers.

Detection of said reporter or marker molecules may be effected by methods known in the art. For example, antibody binding, fluorescent measurement or bioluminescent measurement as well as measurement of products obtained by enzymatic action of enzymes encoded by the reporter genes may be determined. In addition, the reporter or marker molecule may be a gene encoding a cell surface marker or selection marker. For example, the reporter or marker molecule may be the gene encoding the Firefly luciferase or a gene encoding GFP or other fluorescent molecules, in particular, polypeptides like TurboGFP, morange, tdTomato, DsRed-Express, mCherry, DsRed-E5.

Thus, selecting appropriate reporters should allow a precise, specific and faithful non-invasive real-time monitoring of the IFN response in living animals and thus facilitate tracing its spatio-temporal kinetics in vivo for the first time. Moreover, the generated mouse model allows to identify cell types responding to type I IFNs and thereby exhibiting IFN effects after infection with different viruses through various routes. Cells that successfully respond to IFN constitute a site of antiviral status.

In another aspect of the present invention, a method for the determination of molecules altering the function of interferon is provided. That is, said method for the determination of molecules or compounds altering the function of interferon comprises the step of a) providing a cell system according to the present invention; b) providing a molecule or compound to be tested; c) incubating a sample of the cell system according to the present invention in the presence and another sample of the cell system in the absence of the molecule to be tested; d) determining the ability of said molecule or compound to be tested for altering the function of interferon by determining the reporter or marker molecule present in the cells of the cell system according to the present invention compared to the sample of cells incubated in the absence of said molecule or compound; based on the determination of the reporter or marker molecule determining molecules altering the function of interferon in said cell system.

The method according to the present invention is particularly suitable for screening molecules, in particular, large molecule arrays to identify molecules altering the function of interferon, e.g. in high throughput screening (HTS). On the other hand, said method is suitable for validation of known compounds.

Preferably, the cell system useful in the method according to the present invention is a transgenic rodent, in particular, a transgenic mouse or, alternatively, a transduced cell line. Particular preferred the cell system is the cell line NIH3T3 Mx2-Luc2 or the cell line NIH3T3 Gbp2-Rluc deposited with the DSMZ, Braunschweig, Germany under the Budapest Treaty.

The method according to the present invention allows the identification or the screening and/or identification of agonists or antagonists of type I or type II interferon. In this connection, it is noted that the term "agonists" encompasses also enhancer molecules.

Further, the method according to the present invention allows the quantification of type I interferon, in particular, the quantification of type I or type II interferon in biological fluids. Thus, the present invention relates to a method for measuring interferon in a sample comprising the step of a) providing a cell system according to the present invention; b) incubating a probe containing interferon with the cell system and c) determining the amount of interferon present in said probe to be tested. Preferably, quantification is effected by running examples in parallel using samples containing known interferon concentrations allowing standardising and calibrating the measurement. Thus, it is possible to quantify the amount of interferon present in the sample to be tested.

The system and method according to the present invention are particularly useful for measuring interferon concentrations due to the superior specificity and sensitivity of the system. As demonstrated in the examples, said system is superior over known systems based for example on antiviral interferon assays.

As mentioned before, the method and system allow screening of modulators of the interferon system. As a result of the present approach of screening for modulators of the interferon system naturally occurring peptides have been identified. In particular, a cyclic peptide having the general formula I has been identified demonstrating an inhibitory activity on the interferon system.

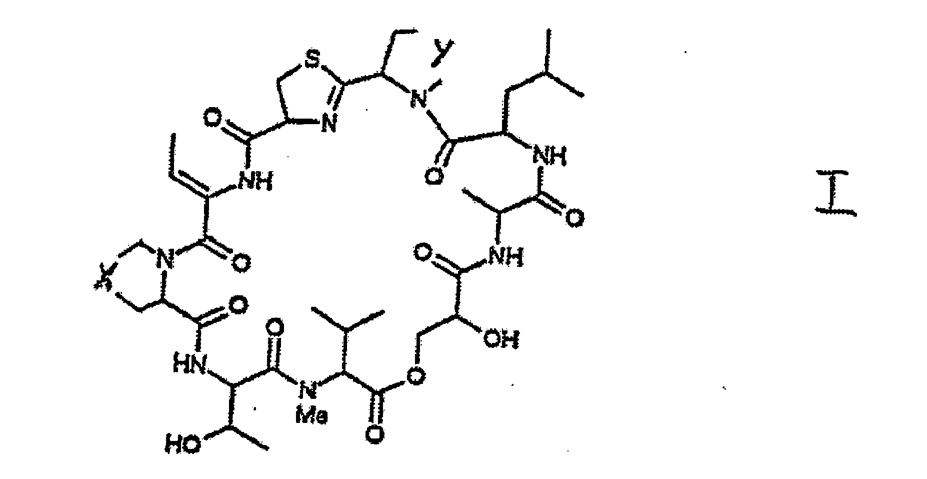

Thus, in another aspect, the present invention provides an antagonist of the interferon system. Said antagonists or conjugates thereof and salt or solvates thereof are particularly useful for the prevention or treatment of septic shock syndrome or autoimmune diseases. In particular, said molecules acting as antagonists of the interferon system are suitable for the prevention or treatment of the systemic and organospecific autoimmune diseases, in particular, of the systemic lupus erythematosus and diabetes mellitus.

Hence, the present invention relates to the use of at least one of the cyclic peptides according to the general formula I 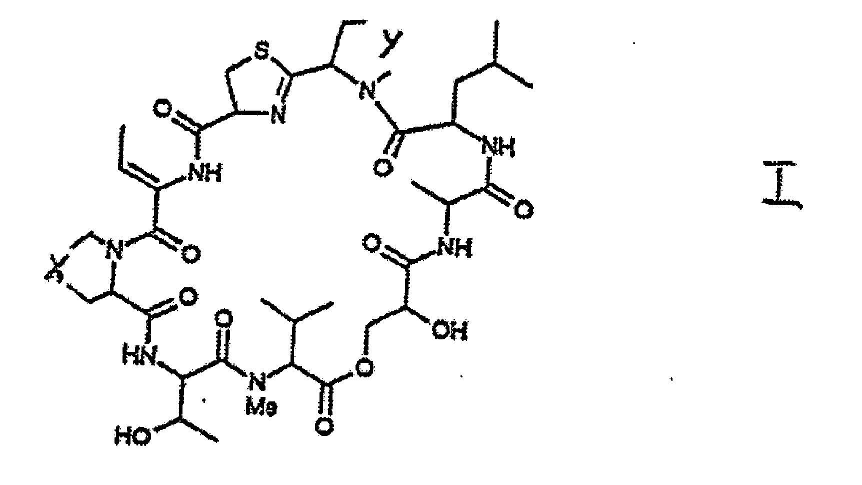
wherein X represents a methylene or an ethylene group and Y is an ethylene or a methylmethylene group,
or conjugates thereof, and salts or solvates thereof,
for the preparation of a pharmaceutical for the treatment or prevention of septic shock syndromes and autoimmune diseases.

Preferably, the cyclic peptide is any one of the Vioprolides shown below:
Vioprolide A 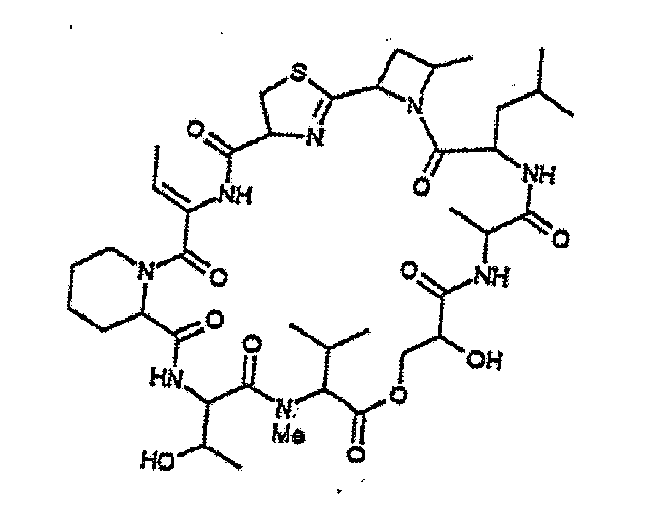
Vioprolide B 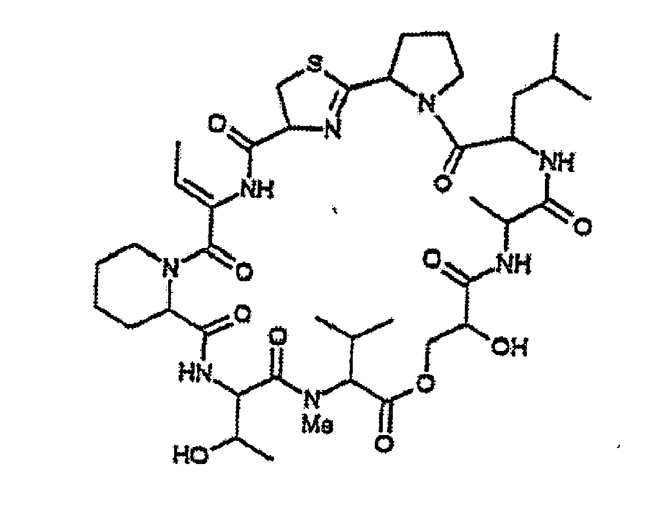
Vioprolide C 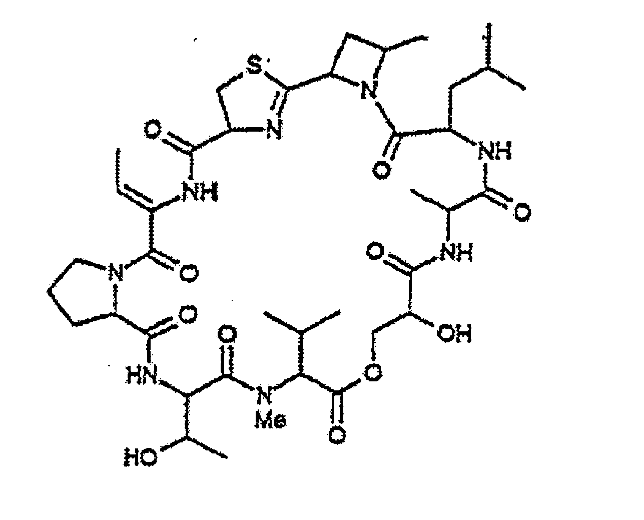
Vioprolide D

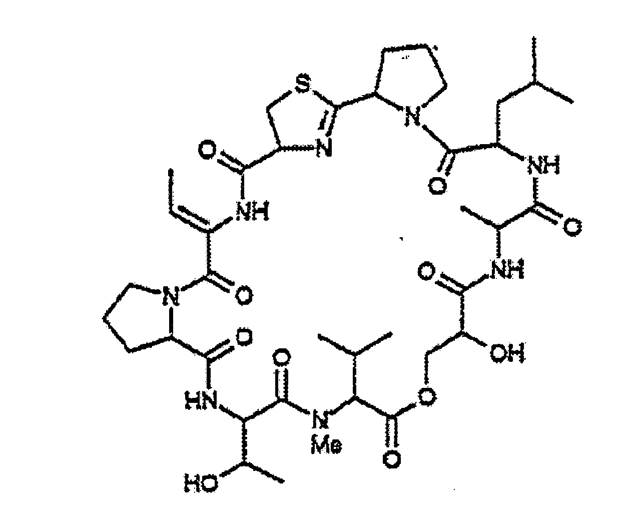

The cyclic peptides according to the present invention are known to have low toxicity.

That is, the cyclic peptides, i.e. the Vioprolides, are extremely useful for inhibiting interferon type I or type II mediated diseases, disorders or conditions. Thus, the use of the cyclic peptide in pharmaceutical preparations and in the treatment or prevention of diseases where interferon type I or type II play a detrimental role is envisaged, in particular for the treatment of autoimmune diseases and septic shock syndrome.

As used herein, the term "conjugate" refers to compounds comprising a conjugate moiety and a compound moiety. The conjugate moiety aims to increase the applicability of the residual compound. The conjugate moiety of the conjugate according to the present invention is a covalently bonded, physiologically tolerated conjugate moiety, which is suitable for converting the cyclic peptides into an even more water-soluble form. For example, the conjugate moiety can be a polymer, a dextran, a sugar, a polyvinylpyrrolidone, an alginate, a pectin or collagen. The conjugate moiety is characterized in that it provides good water solubility and is not immunogenic.

The conjugate moiety of the cyclic peptide conjugate claimed herein, is in a preferred embodiment, a conjugate moiety containing at least one polyalkylene glycol unit of the formula:

X₁-[(CHR₁)ₓ-O]ₙ-(Z)_{y}-

where
X₁ is hydrogen or a hydrocarbon which may contain heteroatom(s);
Z is a divalent linkage group, such as C=O or CHR₁;
R₁ is independently any one of hydrogen, OH, OR₂ or CO-R₃;
R₂ is independently any one of hydrogen or C₁-C₆ alkyl;
R₃ is independently any one of hydrogen, OH, OR₂ or NR₄R₅;
R₄ and R₅ are independently any one of hydrogen or hydrocarbon which may contain heteroatom(s) and which may form a ring;
n is an integer of 1 to 100;
x is independently an integer of 1 to 10;
y is an integer of 0 to 10.

Preferably, n is an integer of 2 to 50, like 2 to 10, in particular 3 to 5.

In another embodiment, x is preferred an integer of 2, 3, or 4, in particular 2.
y is preferred an integer of 1 to 5, in particular, 1 to 3, in another preferred embodiment, y is 0.
X₁ is preferentially OR₆, N(R₆)₂, SR₆ or COOR₆, wherein each R₆ is individually hydrogen, benzyl or C₁-C₆ alkyl, preferably a C₁-C₆ alkoxy group, like a methoxy, ethoxy or propoxy group.
R₁ is preferably a hydrogen atom.

Thus, the polyalkylene glycol unit mentioned above may preferably contain subunits of ethylene glycol, propylene glycol or butylene glycol or combinations thereof. The chain length of each of the polyalkylene glycol units may be in the range of 1 to 100 subunits, preferably, 2 to 50 subunits, like 2 to 10 subunits, particularly in the range of 3 to 5 subunits.

Particularly preferred is the conjugate moiety a methoxypolyalkyleneglycol-carbonyl-residue wherein the alkylene moiety is an ethylene or propylene moiety.

Hence, preferably the conjugates are in a pegylated form to increase the solubility in hydrophilic solvents and hydrophilic environment. Furthermore, the conjugate moiety allows protecting the compound moiety against enzymatic degradation, structural modification due to change of the pH, mechanical removal, etc. Thus, primarily the stability of the compound is increased. Another beneficial effect of conjugation is to increase the retention time in the individual, e.g. to delay the renal excretion, while being well-tolerated, e.g. being non-immunogenic, by said organism.

Specifically, the conjugate moiety comprises at least two chains having polyalkylene glycol units. That is, the conjugate may be a branched compound wherein each arm contains a polyalkylene glycol unit. Particularly preferred are conjugate moieties wherein the polyalkylene glycol unit is a polyethylene, polypropylene or polybutylene glycol unit.

As used herein, the term "pegylated" refers to the conjugation of a compound moiety with conjugate moiety(ies) containing at least one polyalkylene unit. In particular, the term pegylated refers to the conjugation of the compound moiety with a conjugate moiety having at least on polyethylene glycol unit.

### Formulations and routes of administration

Therapeutic formulations of the polypeptide or conjugate of the invention are typically administered in a composition that includes one or more pharmaceutically acceptable carriers or excipients. Such pharmaceutical compositions may be prepared in a manner known per se in the art to result in a polypeptide pharmaceutical that is sufficiently storage-stable and is suitable for administration to humans or animals.

### Drug form

The cyclic peptide or conjugate of the invention can be used "as is" and/or in a salt form thereof. These salts or complexes may by present as a crystalline and/or amorphous structure. The pharmaceutical composition for use in connection with the invention can be formulated as neutral or salt forms. Pharmaceutically acceptable salts include those formed with anions such as those derived from hydrochloric, phosphoric, acetic, oxalic, tartaric acids, etc., and those formed with cations such as those derived from sodium, potassium, ammonium, calcium, ferric hydroxides, isopropylamine, triethylamine, 2-ethylamino ethanol, histidine, procaine, etc.

In order that the invention may be readily understood and put into practically effect, invention will now be described by way of the following non-limiting examples.

### Examples

### Example 1 Synthesis of BAC and Plasmid

Reporter constructs were generated by inserting fragments of the Mx1 or Mx2 promoter into the Firefly luciferase containing pGL3 basic vector (Promega). However, used constructs did not show strong inducibility of luciferase expression after IFN-stimulation (Figure 1). In addition, high basal promoter activities were noticed for both, Mx1 and Mx2 reporter plasmids (data not shown). Since a strong inducibility of the promoter was the key for sensitive measuring of IFN activity, several strategies to augment inducibility of the used Mx promoter constructs were followed. First, promoter regions of Mx2 were extended from previous 0.75 kilo bases (kb) to 1.5 or even 3 kb. Though basal levels could be decreased (data not shown), responsiveness to IFNs got simultaneously weaker. Therefore a different approach was pursued by generating artificial promoter constructs. The 0.75 kb Mx2 promoter construct, which gave best results so far, was used for insertion of additional interferon-stimulated response elements (ISREs). This yielded slight signal increasement after IFN-stimulation, i.e. 5-fold induction compared to uninduced cells. However, it neither seemed to be sufficient for further use in cell culture nor for generation of transgenic mice. The quest for optimal reporter constructs was therefore continued. The next approach to establish optimal reporter constructs was the combination of promoter regions of both Mx1 and Mx2, thereby doubling the amount of ISREs controlling IFN-induced luciferase expression and resulting in 4 of these elements driving transcription of the reporter. With this approach inducibility by IFN could be increased to 6-fold compared to basal signal (Figure 1).

A precise regulation and correct spatial and temporal expression of the reporter is crucial for analysis of IFN responses in cell culture as well as living systems. Therefore bacterial artificial chromosomes (BAC) which offer several advantages were exploited. These vector systems based on the F episome of E. coli can accommodate up to several hundred kb of foreign DNA. BACs are propagated in low copy numbers in recombination deficient strains of E. coli and moreover are quite stable. Due to different recombination systems manipulation of BACs performed in bacteria is well established.

Above all, due to its large size all cis- and trans-regulatory regions of a given gene are usually located on a BAC construct. By virtue of the different genome projects, several BACs harbouring the murine Mx2 locus on chromosome 16 are available and by screening the silicon genome of the joint project Ensembl (http://www.ensembl.org/Mus_musculus) one BAC harbouring the Mx2 gene was selected. Since no information about cis-elements of the Mx2 gene was available, attention was put on choosing a BAC where this IFN-inducible gene is centred on its insert and the 5'- and 3'-flanking DNA exceed 50 kb.

For generation of the reporter BAC, a precise and efficient recombination system for use in bacteria was applied. In this procedure the open reading frame (ORF) of the murine Mx2 gene was replaced by a cassette harbouring the reporter gene of choice (Firefly luciferase or tdTomato) and an FRT (FLP recognition target) flanked antibiotic resistance gene ( kanamycin/neomycin phosphotransferase). For positive selection in bacterial as well as mammalian cells, a dual promoter element combining a prokaryotic with the eukaryotic phosphoglycerate kinase (PGK) promoter for driving expression of the antibiotic resistance gene was constructed. The recombination strategy as well as the replacement cassette is depicted in Figure 2.

### Mutagenesis of Bacterial Artificial Chromosomes (BACs)

BACs harbouring the genomic mouse sequence of interest were ordered at BACPAC Resource Centre (http://chori.org/BACPAC) from the RPCI-23/24 Roswell Park Cancer Institute library. For modification of BACs by homologous recombination ET/Red-Cloning system was performed using the λ-phage recombination enzymes redα, redβ and redγ. First, bacteria carrying the BAC were made electro-competent and transformed with the pKD46 plasmid. pKD46 codes for the λ-phage recombination enzymes and has a temperature sensitive origin of replication (ori). While the plasmid was replicated at the permissive temperature of 30°C, the plasmid was lost when cells were cultivated at the non-permissive temperature of 43°C. E.coli cells containing the BAC and pKD46 were incubated at 30°C on agar plates with ampicillin (pKD46) and chloramphenicol (BAC). The expression of the λ-recombination operon was induced by addition of 1% arabinose to the culture medium when cells were in their exponential growth phase (reaching OD 0.5-0.8); cells were made electro-competent again.

In parallel, PCR was used to amplify the recombination cassette as shown in Figure 2. The oligonucleotide primers were designed so that the PCR product contains the reporter gene (tdTomato, Luc+, Luc2) as well as the FRT-flanked selection cassette. Primers were designed to have 50 bp of overlapping sequence homologous to the target sequence in the BAC. In order to eliminate residual plasmid DNA present in the PCR sample, the DNA was digested with 20 to 40 U Dpnl for 2 h at 37°C. Dpnl recognizes methylated sequences, thus plasmid DNA was digested while PCR products stay intact. The DNA was again purified using the QiaQuick kit. Subsequently, 300 ng of the purified PCR product were electroporated into DH10B cells containing the BAC as well as the pKD46 plasmid, containing the λ phage recombinase enzymes. In these cells the λrecombination operon was induced (30°C) so that homologous recombination could take place. Cells were plated on LB agar plates with chloramphenicol (BAC backbone) and kanamycin (insert) at 43°C to eliminate pKD46. Loss of pKD46 was tested by ampicillin-sensitivity. For analysis of correct homologous recombination events recombinant BACs were subjected for PCR as well as digestive analysis.

The linear reporter cassette was generated by polymerase chain reaction (PCR) using primers with 50 nucleotides extensions homologous to the 5'and 3'sites of the Mx2 ORF, respectively. Replacement through homologous recombination is mediated by enzymes of the bacteriophage λ (Red-γ, -β and -exo) which are provided by a low copy plasmid with arabinose-inducible promoter driving expression of these genes. Primarily, this plasmid encoding the recombination activities was introduced into bacteria harbouring the BAC RP24-71I6 and transient expression of the recombination enzymes was induced by addition.of arabinose. Subsequently, the reporter gene cassette produced by PCR was imported by electroporation. Bacteria with successful recombination were selected using the drug-selectable marker kanamycin and the site of recombination was checked by restriction analysis, PCR and sequencing.

### Example 2 Comparison BAC Plasmid

In a first attempt, the generated reporter BAC harbouring Firefly luciferase under control of the Mx2 promoter as well as the shorter promoter reporter construct with 0.75 kb of the Mx2 promoter region driving expression of Firefly luciferase were used. Hereby, differences between plasmid and BAC-based constructs were analyzed and evaluated for further use as appropriate IFN responsive reporters. Therefore, both reporter constructs were introduced into the genome of NIH/3T3 cells by electroporation, since this result in transfection of one to two copies per cell and thus allows comparison of gene expression. Thus, cell pools of murine fibroblasts with stable integration of the plasmid- or BAC-based IFN-reporter were compared. IFN induced luciferase activity from BAC transfected cells was tremendously higher, i.e. 129-fold compared to 3-fold induction levels of the plasmid-based reporter (Figure 3A). Furthermore, analysis of reporter background levels quantified as relative luminescence units (RLU) related to protein levels showed a stronger signal of unstimulated cells that had incorporated the shorter promoter construct (Figure 3B). It can therefore be concluded, that the used BAC-based reporter shows much stronger inducibility by IFNs and more over exhibit lower basal expression levels. The BAC-based reporter system therefore meets two major criteria for its further use as an optimal IFN responsive reporter.

Moreover, kinetics of an IFN response of these cells was analyzed following reporter signals after stimulation with 500 U/ml of IFN-β for different time points. As can be seen in Figure 6C the BAC-based reporter cells exhibited much stronger induction levels. Already eight hours after stimulation a significant increase in luciferase activity (for p=0.008) was observed. In contrast, first significant signals of the plasmid-based reporter (for p=0.017) were detected only twelve hours after IFN-stimulation. The climax of both reporter cell lines was noticed between 16 and 24 hours after stimulation. Putting attention on the later time points of IFN exposure it can be stated that the decrease in the IFN response is much stronger in the BAC-based system as a more than 50% reduction in luciferase activity between day one and three after IFN-stimulation was detected.

Cell pools of NIH/3T3 stably transfected by electroporation with the BAC Mx2Luc ("BAC") or the plasmid pGL3-Mx2P 0.75kb ("Plasmid") were used for comparison of IFN responses. In figure 3A the results are shown as fold induction resembling RLU of IFN-β-stimulated (500 U/ml) compared to uninduced cells. As control the promoterless pGL3-basic vector (Promega) was used. Figure 3B shows RLUs calculated to one µg of protein detected by BCA assay. RLUs were assessed in both unstimulated and IFN-I3-stimulated (500 U/ml) cells containing the plasmid (pgL3Mx2P 0.75kb) or BAC (Mx2Luc) based reporter. In figure 3C cells were assayed for their kinetic to IFN-β stimulation (500 U/ml) and response was followed over time (0 - 96 h) by measuring luciferase activity. Fold induction resembles detected RLUs compared to uninduced state. P-values were calculated by student's t test (* p=0.008, **p=0.017). Data represent mean ±SD of two experiments (n=2)

### Example 3 Bioassay using a cell system according to the present invention

The general purpose of any bioassay is to quantify a functional response in a living system. In terms of cytokines it is generally accepted that their potency should not be expressed in molar or mass concentrations as this describes the physical quantity and not the most important feature - their biological activity. For instance, the potency of single cytokine preparations may vary significantly due to storage conditions without any change in molarity or mass. In respect to biological assays for measuring IFN concentrations several activities like their antiviral or antiproliferative ones have already been employed. Here another approach for quantifying IFN concentrations was addressed by analyzing transcriptional induction of a reporter gene under control of an IFN-inducible promoter. By using firefly luciferase as reporter IFN concentrations of unknown samples can be determined by correlating the level of luciferase enzyme activity to an employed IFN standard.

For measuring type I IFNs BAC-based reporter constructs have been used. BACs harbouring the cDNA of Firefly luciferase 2 (luc2) under control of the Mx2 promoter were engineered, NIH3T3 Mx2-Luc2. The change from Luc+ (pGL3 basic, Promega) used in the before mentioned reporter constructs to the next generation luciferase (pGL4.10, Promega) was performed, as Luc2 theoretically promised to achieve higher signal intensities and therefore might also be more practicable for further in vivo imaging experiments. This newly generated BAC was electroporated into the murine fibroblast cell line NIH/3T3.

Namely, NIH/3T3 cells were electroporated with recombinant BAC Mx2Luc2 and cell clones stably expressing the BAC were obtained by G418-selection. Cells were expanded and subjected for further analysis of their response to IFN-β. Cells were seeded on 6-well plates and stimulated wit 500 U/ml of IFN-β for 24 hours (+IFN) or left untreated (-IFN). Luciferase Activity was analysed by luminometric assay as described in Materials and Methods (n=2). In figure 4A, relative luciferase units (RLU) were calculated to protein amount, which was obtained by BCA-assay. Results of each obtained cell clone are displayed. In figure 4B fold induction of RLU obtained after IFN-stimulation compared to untreated cells is displayed. Surprisingly, a wide variation of both background luciferase activity and inducibility between generated cell clones was apparent. Out of twelve showing resistance to G418 and sensitivity to IFN-β three were taken for further analysis. These cell clones, showed highest inducibility by type I IFNs and low background levels in the uninduced state. A 1,378-, 7,913- and 1,220-fold induction after stimulation with 1,000 U/ml IFN-β compared to reporter signals of untreated cells was calculated respectively. All other clones gave higher levels of constitutive expression and/or lower inducibilities. After preliminary data were obtained for selecting the optimal cell clone for use as an IFN bioassay, clone #3 was taken for further analysis, especially with regard to its high inducibility.

In the following this clone is referred to as IFN-reporter cell line for the sake of convenience and understanding.

Further experiments demonstrated that the system showed a dose-dependent response to various type I interferons, reflecting authentic regulation of the Mx2 gene and, thus, of IFN activity.

### Example 4 Quantification of IFN

### Comparison of Mx2-Bioassay to an Antiviral Assay

The most commonly and widely used assays for measuring IFN concentrations are based on the antiviral activities of these proteins. In these setups the inhibitory effect of IFNs on viral propagation or replication is detected and quantified. One assay routinely performed in our department is based on inhibition of the cytopathic effect (CPE) that results from infection of unstimulated murine fibroblasts with vesicular stomatits virus (VSV). Thereby, the concentration is calculated by comparing inhibition of cell lysis to an IFN reference standard. However this assay has several drawbacks as it is tedious to perform and someone needs special expertise and authorization in virus-handling and analysis of results. Besides, for precise quantification of cell lysis staining methods, which often result in removal of cells due to several washing steps, have to be performed. Therefore this new generated bioassay based on the reporter gene induction driven by the Mx2-promoter serves as an alternative and appealing approach for quantification of type I IFNs.

In the following, samples with unknown IFN-concentrations were applied on both the newly established Mx2-bioassay and the antiviral assay and results were compared. Since the obtained samples derived from supernatants of NDV-infected murine embryonic fibroblasts (MEF) and any additional effects of remaining virus particles on the reporter cell line had to be avoided, ultracentrifugation was performed. Then, the Mx2-based reporter cells as well as LMTK-cells used for the antiviral assay were stimulated with 100 pi of each sample. For each assay and IFN-concentrations were calculated.

Five samples of unknown IFN-concentrations were employed to compare the new established Mx2-bioassay with the common used antiviral assay to determine IFN-concentrations. In figure 5A an antiviral assay was performed as described in the following.

### Antiviral Assay for Quantification of IFNs

Staining solution 5 g cristal violet, 143 ml formaldehyde, 500ml ethanol, 5 g NaCl8, in 1 L H2O IFN concentrations in cell culture supernatants and serum were determined by an antiviral assay using mouse LMTK⁻ cells and vesicular stomatitis virus (VSV) as challenging virus Cells were plated on microtiter plates (1x106 cells/plate) and after one day stimulated with supernatant and IFN-standard of known concentration. Therefore, samples were applied on cells of first row, and serial dilutions (1:1) were performed until last row. One day later, cells were infected with VSV (1.7x104pfu/ml) in DMEM3+ (+5% FCS) and cell lysis was analysed by microscopy and living cells were stained with 100µl or a crystal violet staining solution, which was incubated for 30 min at room temperature. Afterwards plates were washed three times with water and left for drying. For analysis of Vioprolide A effects on IFN-induced antiviral activities nearly same procedure was done, except that MLT-1 cells were used and VSV was applied in several dilutions (1.7x103, 1.7x104, 1.7x105 pfu/ml).

Samples were applied onto LMTK⁻ cells in two different dilutions: 1.row: undiluted, 2.row: 1:10 diluted in culture media. For control (Ctrl) 500 U/ml IFN-β were applied on cells. Image represents results obtained by two independent experiments. In figure 5B calculated concentrations of samples (A-E) obtained by antiviral assay and the Mx2-bioassay are displayed. To determine IFN-concentrations with Mx2-bioassay the IFN-reporter cells, containing the recombinant BAC Mx2Luc2, were seeded on 24-well plates (2.104 cells / well) and incubated with 20% of IFN-samples overnight. For quantification a standard curve of different IFN-β concentrations (0, 10, 25 ,50, 100 and 250 U/ml) was additionally applied. Unknown IFN-concentrations were calculated by correlating obtained RLUs to employed standard curve. C IFN-β concentrations [pg/ml] obtained by IFN-β specific ELISA (PBL).

Strikingly, great differences between both assays became evident (Figure 5B). For impartial evaluation of both assays IFN-concentrations were additionally detected by an ELISA specific for IFN-β (Figure 5C). Although the IFN protein mass can hardly be compared to IFN activities some issues arose and can be discussed. First of all, no activities were detected in sample A by all three assays, which indicate that false-positive results can hardly be obtained. Second, with the ELISA assay IFN-β amounts in sample B could be detected (64 pg/ml). This agrees with the Mx2-bioassay which also verified small amounts of IFNs (3 U/ml), though levels were outside the linear range for precise calculations. However, no IFN was detectable in this sample with the antiviral assay (Figure 5A). Intriguingly are results obtained for sample E. Here, data resulting from the antiviral assay suggested lower IFN levels in E compared to D. This greatly differs from those obtained by the Mx2-bioassay as well as the ELISA. Both indicated much higher IFN levels in sample E than in D. Since endogenously produced IFN was measured and IFN-β amounts were calculated for these two samples to be 2,310 and 3,120 pg/ml, respectively, also higher levels of active IFN in E can be assumed.

Thus, detection limits in lower as well as higher IFN levels when using the antiviral IFN assay became evident. In addition to already mentioned advantages of the newly developed Mx2-bioassay a higher sensitivity compared to the commonly used antiviral assay could be proven.

### Example 5 Screening of potential inhibitors

In a first step, a library of natural components of the Helmholtz centre for infectious diseases has been screened. As a promising compound, the natural compounds of Vioprolides produced by the myxobacterium Cystobbacter violaceus have been identified. These compounds have been characterized further on their inhibitory activity.

For further analysis of the inhibitory effect of Vioprolide A on the IFN system reporter cells harbouring the Mx2Luc2-BAC were stimulated with different concentrations of Vioprolide A two hours prior to IFN-β treatment. After overnight incubation luciferase activity was determined and calculated according to protein levels to scale with the antiproliferative effects of Vioprolide. An inhibitory effect of Vioprolide A on the type I IFN response could be observed for all concentrations tested. Strongest inhibition was detected with 100 ng/ml of Vioprolide A, revealing a dramatic decrease of Mx2-driven luciferase activity down to background levels (Figure 6A). The median inbibitory concentration (IC50), which reduces IFN-induced responses by 50%, was calculated to be 10 ng/ml of Vioprolide A.

In figure 6A type I IFN reporter cells, NIH3T3 Mx2-Luc2 were pretreated with different concentrations of Vioprolide A (-, 5, 10, 30, 50 or 100 ng/ml). After pre-treatment for two hours cells were stimulated with IFN-β (500 U/ml) in the continuous presence of the indicated Vioprolide concentrations. In figure 7B type I IFN reporter cells were pretreated with IFN-β (500 U/ml). After four hours cells were additionally stimulated with different concentrations of Vioprolide A (-, 5, 10, 30, 50 or 100 ng/ml). Untreated cells were used as control (Crtl). Luciferase activity was measured after 24 hours and relative luminescent units (RLU) were calculated to one µg of protein detected by Bradford assay. Mean values ± SD are displayed (n=2). * p<0.05 and **p<0.001 for Student's t test compared to IFN stimulated cells without Vioprolide treatment.

To confirm these data, cells which have stably incorporated the recombinant reporter BAC Mx2Tom harbouring the red fluorescent protein tdTomato under control of the IFN-inducible Mx2-promoter were used. This allowed assessment of the Vioprolide A effect on the IFN system on a single cell level. Thus, cells were stimulated with IFN-β as wells as different concentrations of Vioprolide A and expression of tdTomato was analyzed after overnight incubation using flow cytometry. Again, a dose-dependent inhibitory effect of Vioprolide A on the reporter expression could be observed. Thus, it can be stated that Vioprolide A exhibits a negative effect on type I IFN responses determined by a decreased activation of the IFN-inducible Mx2-promoter.

Next, the effect of Vioprolide A on previously activated type I IFN signalling was addressed. This was performed to exclude a direct inhibitory interaction of this natural compound with IFN molecules or their cognate receptors type I IFN reporter cells were therefore stimulated with IFN-β four hours prior to treatment with different Vioprolide A concentrations. After overnight incubation cells were harvested and luciferase activity was assayed. Strikingly, application of 100 ng/ml of Vioprolide again led to a significant reduction of reporter expression, indicating a strong impact of this peptide also on prior activated IFN-signalling cascades. A first significant reduction of luciferase activity (for p<0.05) could be visualized with 30 ng/ml Vioprolide A (Figure 6B). The IC50 was identified to 20 ng/ml. Thus, Vioprolide A does not interact with type I IFN proteins or their cognate receptors but seems to act on their downstream signalling cascades by blocking ISG expression.

In addition, Vioprolide A might also modulate type II IFN responses. Hence, this hypothesis was also validated and a reporter system responsive to type II IFN was used. Hereby, expression of Renilla luciferase is driven by the type II IFN responsive promoter of the Gbp2 gene obtained using a BAC containing the GBP2 gene locus and following the method described above. In this experiment, reporter cells, NIH3T3 Gbp2-RLuc were seeded on 24-well plates, stimulated with 500 U/ml IFN-γ and one hour later incubated with different concentrations of Vioprolide A. Afterwards the IFN response was analysed by quantifying Renilla luciferase activity. Since this assay was performed on a microtiter format, quantification of protein amounts by former used BCA as well as Bradford assay was impossible. Therefore, incubation time of Vioprolide A was kept as short as possible, i.e. around ten hours, to obtain comparable cell counts. All concentrations tested showed a decreased response to type II IFN (Figure 7). Though luciferase activity could not be calculated to protein levels and the blockade might not be that dramatic, these results emphasize the influence of Vioprolide A on the type II IFN response. Thus, evidence for a negative influence of Vioprolide A on both type I and type II IFN responses was provided. Furthermore, these data suggest an impact on signalling cascades shared by both type I and type 11 IFN response pathways.

Further experiments demonstrated the reversibility of the influence of Vioprolide A on the IFN system. In addition, Vioprolide demonstrate an inhibitory effect on IFN-mediated antiviral activities.

### Example 9 Generation mouse

### Generation of Transgenic Reporter Mouse Model

To establish a transgenic model organism to study IFN responses in vivo the advantages of BACs for powerful manipulation of the mouse genome were utilized: Transgenes included in such genomic-type constructs are normally correctly expressed according to co-delivery of near-endogenous chromatin environment.

Furthermore, the use of BACs usually ensures inclusion of all regulatory elements required for proper expression. Therefore BAC-based transgenes are less likely to be influenced by position effects compared to smaller ones.

A transgenic mouse carrying the murine Mx2-containing BAC RP24-7116, which had been modified to express the Firefly luciferase reporter gene under control of endogenous regulatory elements of the Mx2 gene locus, was generated. By virtue of its high sensitivity, the virtual absence of background signals and the benefits of luminescence quantification in vivo bioluminescence imaging (BLI) luciferase reporter was chosen for visualising IFN responses in this first trial. This would offer a first overall impression of the spatio-temporal dynamics and, due to special properties of Firefly luciferase, swift changes in the IFN response could be unveiled. For BAC recombineering techniques a FRT-flanked cassette containing a kanamycin/neomycin phosphotransferase gene controlled by prokaryotic as well as eukaryotic promoter elements was needed. However, potential promoter crosstalk between the constitutive active phosphoglycerate kinase (PGK) and the IFN-inducible Mx2-promoter had to be avoided as this might affect the level of reporter gene expression. Therefore, the FRT-flanked selection cassette was excised using site-specific recombination mediated by the Flpase enzyme recognizing these specific FRT-sites.

Namely, for pronuclear microinjection the FRT-flanked selection cassette harbouring prokaryotic and eukaryotic promoters driving expression of the kanamycin/neomycin phosphotransferase gene of the recombinant BACs Mx2Luc2 and Mx2Tom was excised. Therefore, bacteria containing BACs were made electrocompetent and afterwards transformed with the pFlp708 vector, expressing the Flpase enzyme. Cells were grown at 30°C on chloramphenicol (cm, BAC) tetracycline (tet, plasmid) agar plates. Selected colonies were picked and used for inoculation of 5 mL LB-media with cm/tet. Suspension was incubated overnight at 30°C, as this temperature allows plasmid-replication but not expression of the Flpase enzyme. Following day, 10µl of the culture were transferred in 1 ml fresh LB-media and left on shaker for 8-9 hours at 38°C. For correct excision, bacteria were analysed for their sensitivity to kanamycin, as well as by PCR-analysis. The newly generated BAC was named Mx2Luc2≠Neo. Subsequently, transgenic animals were generated by microinjection of the linearised BAC DNA into pronuclei of zygotes. This transgenic approach yielded 32 pubs based on C57BL/6 genetic background. Progenies were screened for BAC-integration by PCR of tail-derived DNA applying two distinct primer pairs. Three founders could be identified and offspring were used for analysis of luciferase activity and continuing validation of their use as faithful reporters of IFN-responses.

Thus, this novel transgenic mouse model allows for the first time non-invasive real-time monitoring of type I IFN responses after different stimuli, achieved by analysis of Mx2-promoter controlled luciferase activity and its visualization through in vivo molecular imaging devices.

Further experiments demonstrated that an induction of transgene expression by IFN-β can be observed and in vivo monitoring of type I IFN responses in mice is possible. In addition, from examination of various organs obtained from mice at different induction stages, the IFN-β dependent luciferase activity in individual organs can be observed.

In further experiments, the reporter activity has been evaluated in different cell types and tissues. Namely, the IFN-responses in BM-derived macrophages and fibroblasts have been examined.

In both cell types, a time- and dose-dependent kinetic of IFN-responses in these cells derived from transgenic IFN-reporter mice was proven. Thus, the mice model is suitable for monitoring IFN response in vivo.

Another experiment demonstrated that also indirect inducers of IFN, like the dsRNA analogue poly (I:C) can elicit an IFN response which can be monitored with the present cell system, the transgenic mouse.

Finally, IFN-responses to viral infections have been monitored using the well established VSV system.

Thus, it can be stated that the generated mouse model is suited for online monitoring of type I IFN responses after viral infections and serves therefore as an appealing system for research on the interferon system and the formation of an antiviral state after intrusion of pathogens.

### Example 12 Employment of Vioprolide in the transgenic mouse model

In an attempt to screen for potential inhibitors of the interferon system the class of Vioprolides, which are secondary metabolites of the myxobacterium Cystobacter violaceus, was found to modulate IFN responses in cell culture systems as identified above. Since Vioprolide A exhibited the highest impact on IFN responses in vitro this peptide was used to examine its effect in living animals. Furthermore, results can also serve for evaluation of the novel type I IFN responsive mouse model for its ability to respond to potential modulators of the interferon system. Since this experiment was based on explorative means to gain a first impression of the reporter mouse model and the impact of Vioprolide A on transgene expression only a small number of animals was used, i.e. one mouse for each stimulation.

For the following in vivo experiment 20 µg of Vioprolide A were employed. This amount was calculated according to a publication appointing the lethal dose of this compound to be 3 mg/kg and moreover to concentrations used for anti-tumour studies Thus, type I IFN reporter mice were intravenously injected with Vioprolide A and subjected for bioluminescent imaging. After one hour mice were additionally treated with IFN-β and responses were assessed after a three-hour incubation time. This period was chosen for analysis as prior experiments identified signal peaks to be between three to six hours after IFN-treatment. Strikingly, Vioprolide A even exerted an influence on the interferon system in vivo (Figure 8). The animal treated with both Vioprolide A and IFN-β exhibited less luciferase activity, assessed by optical imaging, than the one stimulated with the cytokine alone. Furthermore, quantification of light emission revealed signals after IFN treatment to be ten times higher than those obtained after combined IFN- and Vioprolide stimulation.
Though mice emanated of one litter differences in backgrounds, i.e. prior to IFN- but after Vioprolide stimulation, were found to exist. This might result from differences in expression patterns between animals or could indicate an impact of Vioprolide A also on basal reporter expression like it was observed in cell culture. However, these experiments only served for a first impression and experiments need to be followed up with a greater number of animals. Thus, first evidence about inhibitory effects of Vioprolide A on the interferon system in transgenic mice was provided. This impact on both IFN signalling and basal reporter expression will be the subject for further and detailed research. Furthermore, a dynamic response of the IFN-responsive transgene towards modulators could be proven and will also be addressed in comprehensive studies.

In figure 8A female type I IFN reporter mice, which contain the IFN-responsive BAC Mx2Luc2, were i.v. treated with 20µg of Vioprolide A (in 100% EtOH). First image was taken immediately after Vioprolide A treatment. After 1 h mice were i.v. injected with 5.000 U/ml IFN-13 (PBL). Three hours after IFN-treatment second series of images were taken. For control one animal was only treated with Vioprolide A or with IFN, respectively. Bioluminescent images were obtained using the IVIS200 System (Xenogen). Therefore mice were anaesthetised with 2-2.5% isoflurane (Isoflo, Abbott) and i.p. injected with 100 µl of luciferin (30mg/ml in PBS; Synchem OHG). Rainbow scales indicate number of photons measured per second x cm2 x sr. In figure 8B quantification of luminescence was performed using "Living Image" software and average (avg) radiance calculated as photons per second x cm2 x sr is displayed.

## Claims

1. System for the determination of molecules altering the function of interferon (IFN) comprising a cell system at least partially containing exogenous chromosomal DNA, delivered by a recombinant vector containing interferon responsive DNA promoter elements, having a size of at least 3 kb 5' upstream of the minimal interferon responsive DNA promoter elements conferring responsiveness to type I or type II interferon stimulation, operably linked with a nucleic acid sequence encoding a reporter or marker molecule, into cells of the cell system.

2. The system according to claim 1 wherein the recombinant vector is a recombinant YAC or BAC.

3. The system according to claim 1 or 2 for the determination of molecules altering the function of type I interferon.

4. The system according to claim 3 wherein the type I interferon responsive promoter element is selected from the Mx1, and the Mx2 gene locus.

5. The system according to claim 1 or 2 for the determination of molecules altering the function of type 11 interferon.

6. The system according to claim 5 wherein the type II interferon responsive promotor element is the Gbp gene locus.

7. The system according to any one of the preceding claims for the quantification of IFN.

8. The system according to at least one of claims 1 to 7 wherein the cell system is a transduced cell line or a rodent animal, in particular, a transgenic mouse.

9. The system according to at least any one of claims 1 to 8 for in vivo validation or monitoring of the function of interferon.

10. The system according to any one of the preceding claims wherein the reporter/marker molecule is selected from the group consisting of bioluminescent, fluorescent, enzymatic and antigenic reporter genes, and nucleic acid sequences encoding cell surface marker and selection marker.

11. A method for the determination of molecules altering the function of interferon comprising the step of
a) providing a cell system according to any one of claims 1 to 10;
b) providing a molecule to be tested;
c) incubating a sample of the cell system of a) in the presence and in the absence of the molecule of step b);
d) determining the ability of said molecule of step b) for altering the function of interferon by determining the reporter or marker molecule;
e) determining molecules altering the function of interferon in said cell system.

12. The method according to claim 11 for screening and/or identifying agonists or antagonists of type I or type II interferon.

13. A method for the quantification of interferon comprising the steps of:
a) providing a sample to be analysed for the amount of interferon,
b) providing the cell system according to any one of claims 1 to 10,
c) incubating the sample of a) with the cell system of b),
d) determining the reporter or marker molecule;
e) calculating the amount of interferon based on the amount of reporter or marker molecule determined in step d).

14. A cyclic peptide having the general formula I 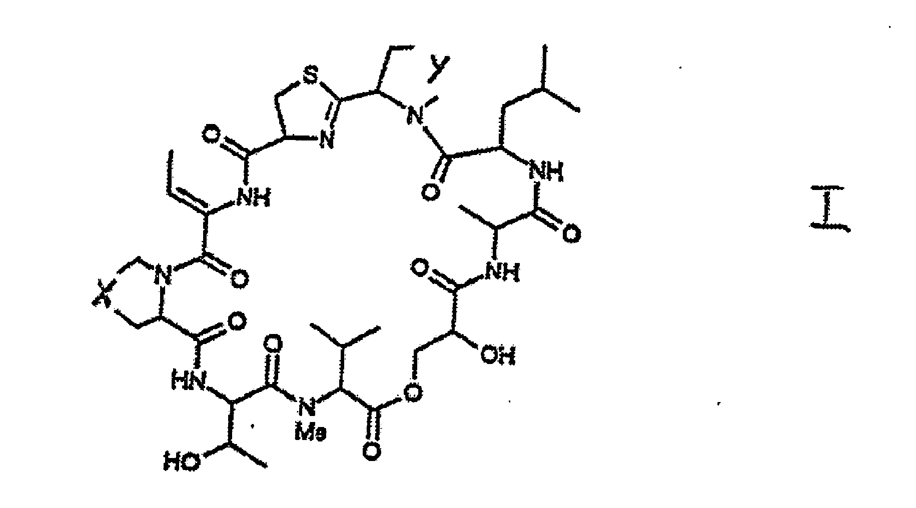 wherein X represents a methylene or an ethylene group and Y is an ethylene or a methylmethylene group, or conjugates thereof, and salts or solvates thereof, for the prevention or treatment of septic shock syndrome, or autoimmune diseases.

15. The cyclic peptide according to claim 14 for the prevention or treatment of systemic and organo specific autoimmune disease, in particular, of systemic lupus erythematosus and diabetes mellitus.
